# EUROPEAN PATENT APPLICATION

(11) **EP 1 246 113 A1**
(43) Date of publication of application: **02.10.2002**
(21) Application number: 01302174.6
(22) Date of filing: 09.03.2001
(51) Int. Cl.: G06F 19/00

(54) **Method & apparatus for delivering healthcare**

(71) Applicant: Pihl Limited, Dublin 8 (IE)
(72) Inventor: Sorensen, Lars, DK-4390 Vipprod (DK)
(74) Representative: Lloyd, Patrick Alexander Desmond

(57) **Abstract**

A healthcare management system integrates medical practitioners, healthcare administrators, patients and educators/students. Patient data is stored in a repository as clinical data with associated patient data. The clinical data is available for medical practitioners, educators and students without the patient data. The system is structured as an application layer accessed by user terminals and separated from the repository by a data access layer which stores details of where, and in what format, data is stored.

## Description

This invention relates to the delivery of healthcare and, in particular, to the organisation of healthcare systems.

In present healthcare systems there is an attempt made at an interchange of information between doctors, patients and healthcare administrators.

Hospitals run many systems which assist in separate areas of the provision of healthcare but there is a lack of any overall cohesion of such systems. For example, a doctors surgery may have an individual patient's prescription history on computerised record but those records are held in isolation; thus, the doctor treating a specific condition in the patient does not have easy access to knowledge gained from the treatment of other patients with the same condition by other doctors. This makes it difficult for the doctor to deliver the best healthcare to the patient. Similarly, the pharmaceutical company which has manufactured the drug does not have general access to the individual patient records and so has difficulty in further developing the drug, for example by finding optimum dosages. At present this requires specific patients to be enrolled in trials which can be very lengthy and produces a narrow spectrum of results.

Demands are increasing from patients who see themselves as employees of healthcare systems and its representatives. Patients are demanding better quality documentation and treatment as well as more information. Furthermore, the number of diagnostic methods and therapeutic opportunities has increased greatly over the last 10 to 15 years leaving the practitioner with access to technical devices and systems he hardly understands or is not even aware of. This can result in millions of lab tests that are unrelated to the actual treatment. It can also result in a lack of insight into, and application of, new and potentially more effective methods of treatment.

Healthcare managers, which include administrators, private healthcare executives, national or local healthcare systems, and insurers, are demanding better financial insight and control over escalating healthcare costs without sacrificing quality care. Physicians are typically not educated in economics or business administration and many of them are not interested in such matters as they are ancillary to their professional objectives.

The medical education and research establishments do not have any way of feeding back in real time, diagnosis and treatment decisions which take place in the medical marketplace, making insight into those decisions difficult at best. The practitioner is also denied real-time access to the latest developments in the field without attending conferences and symposia which can be far away and do not provide immediate feedback to decisions they make on a day to day basis.

Attempts have been made to integrate healthcare core systems but none has been successful. They have tended either to be designed by medical professionals who have not adequately understood information and computer systems or by information technology professionals who have not adequately understood the medical systems they are trying to implement.

There is a need, therefore, in the medical industry, to provide a system which can integrate the various resources and information within the industry so that the quality of healthcare can be improved by proper utilisation of existing data.

According to the invention there is provided a healthcare management system, comprising a plurality of user terminals; an application layer coupled to the user terminals and running a plurality of healthcare related application programs; a repository for holding patient data, the repository storing patient identification data and clinical data separately, whereby clinical data may be retrieved with or without the patient identification data to which it relates; a data access layer arranged between the application layer and the repository for retrieving data from the repository or one of a plurality of further databases external to the system, the data access layer including information regarding the location of data required by the application programs.

Embodiments of the invention have the advantage that they may easily be integrated into existing healthcare system which may already include much of the data that is required by the system.- Thus, to install a system into a hospital or other healthcare system, only the data access layer needs to be designed specific to that hospital.

Preferably, the data access layer comprises a query processor which receives requests for data from the application layer and includes means for retrieving a document describing where to find the data requested and means for retrieving that data and passing it to the application layer. The document retrieved by the query processor may also describe the format of the data to be retrieved.

Alternatively, the data access layer comprises data access objects.

Preferably, the application later models patients as entities owning health track entities. It is preferred to embody the system using enterprise Java beans. A health track is an entity owned by a patient. An activity is an entity related to a single health track. To obtain a full patient record, a Java session bean must instantiate the patient entity related health track entity and activity entities related to the health track entity.

Preferably, each of the user terminals includes a browser.

The terminals may be thin or thick client and present information as HTML or XML documents to users which appear as web pages although they are not.

The invention also provides a healthcare management system comprising a computer network for communicating between a plurality of user terminals and an application system for running a plurality of application programs, the application system including a database storing clinical data and related patient data, and an interface to a plurality of further data stores each containing patient related information; the communications network linking medical practitioners, healthcare administrators, patients and educations establishments and/or students, whereby each have access at least to clinical data stored in the database.

Embodiments of this aspect of the invention have the advantage that they can integrate the clinical, patient, administrative and educational aspects of healthcare using a common pool of data. This enables a more complete interchange of information within a healthcare system. For example, students and educators to have access to up to date clinical data and practitioners to have access to up to date treatment and diagnosis information.

Preferably a neural network is provided which can assist in diagnosing patient conditions or confirming a doctors diagnosis.

Embodiments of the invention will now be described with reference to the accompanying drawings, in which:
Figure 1 is a schematic overview of the components integrated in a system embodying the present invention;
Figure 2 is an expanded version of Figure 1 showing different constituents of each of the components of Figure 1;
Figure 3 shows how the components of Figures 1 and 2 may be provided on various computer networks;
Figure 4 is a view of the various different application pathways in a system embodying the invention;
Figure 5 is a schematic view of a system embodying the invention;
Figure 6 is a flow chart illustrating how patient details may be obtained using the system;
Figure 7 is a data flow diagram illustrating operation of the system;
Figure 8 to 24 are screen shots of various modules at various stages of operation, specifically:

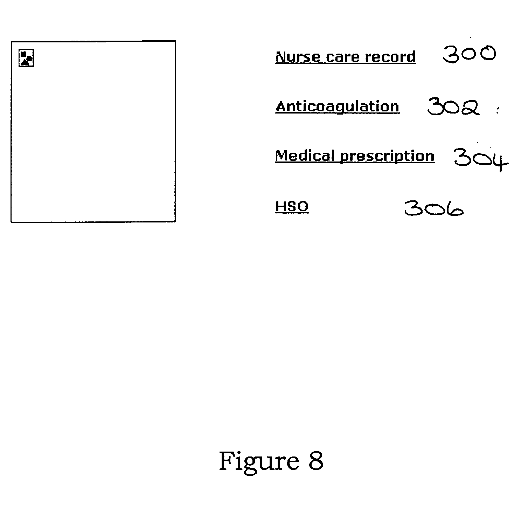

   Figure 8 is an opening screen presented to a user;

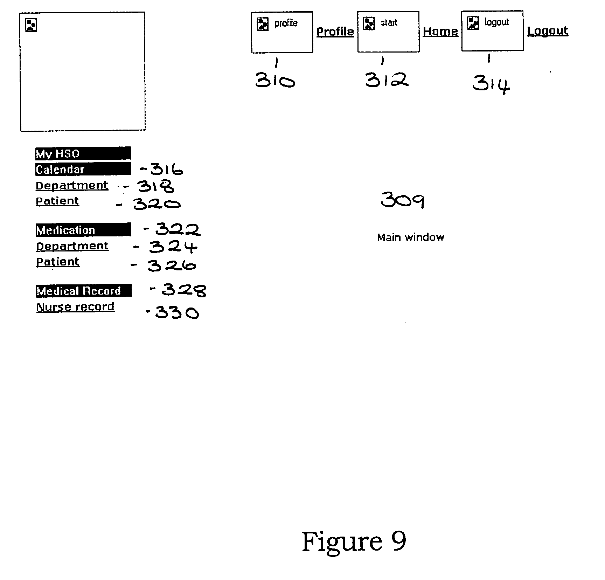

   Figure 9 is a nurse care screen;

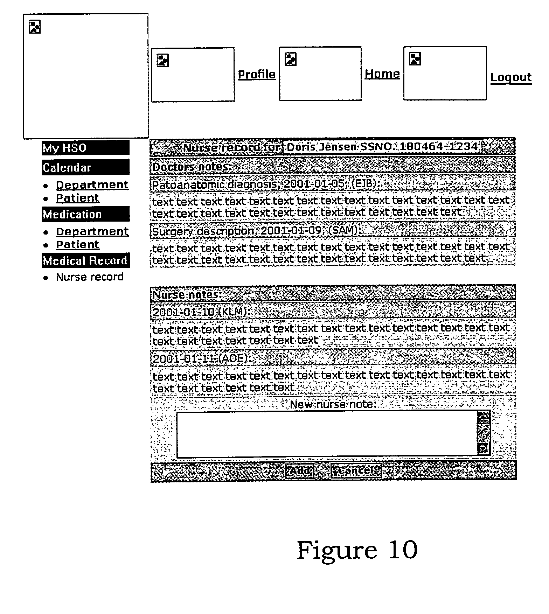

   Figure 10 is a nursing journal view;

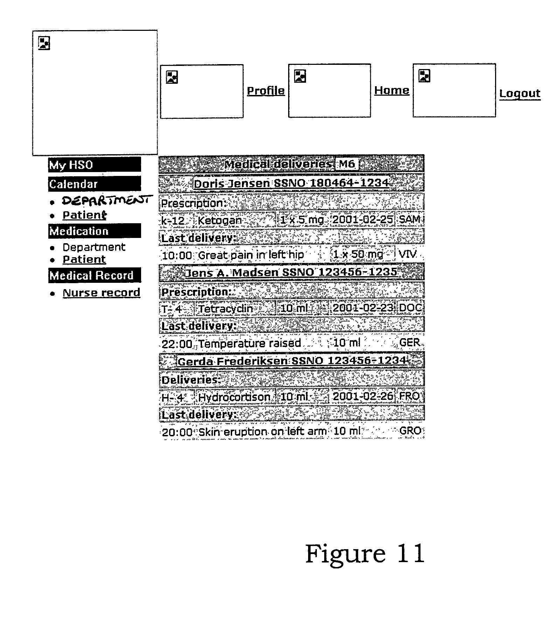

   Figure 11 is a medicine prescription screen for a department or ward;

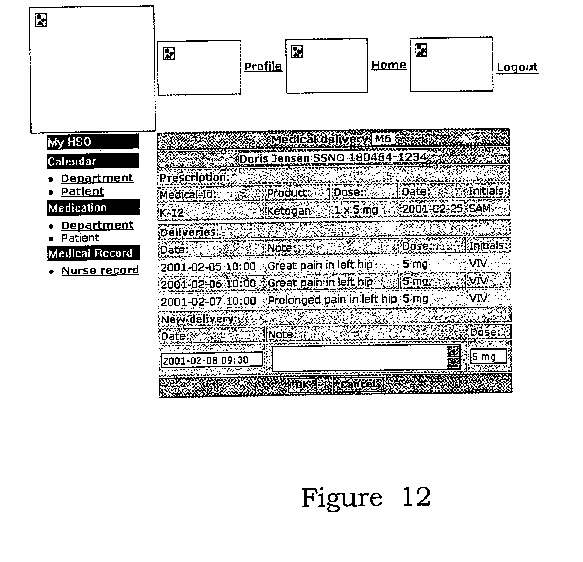

   Figure 12 is a medicine prescription screen for an individual patient;

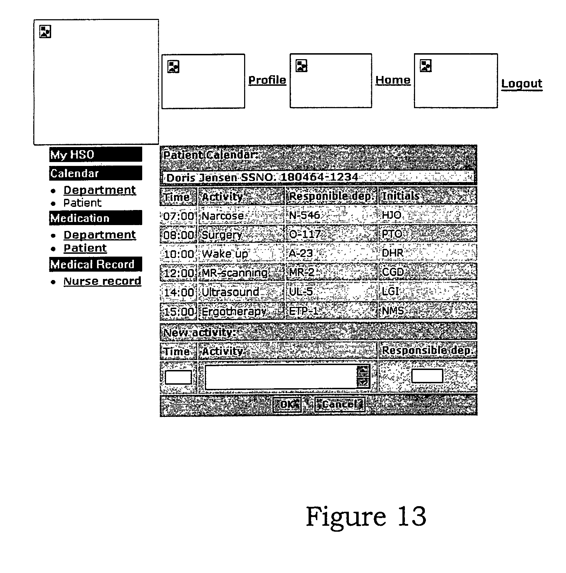

   Figure 13 is a patient calender;

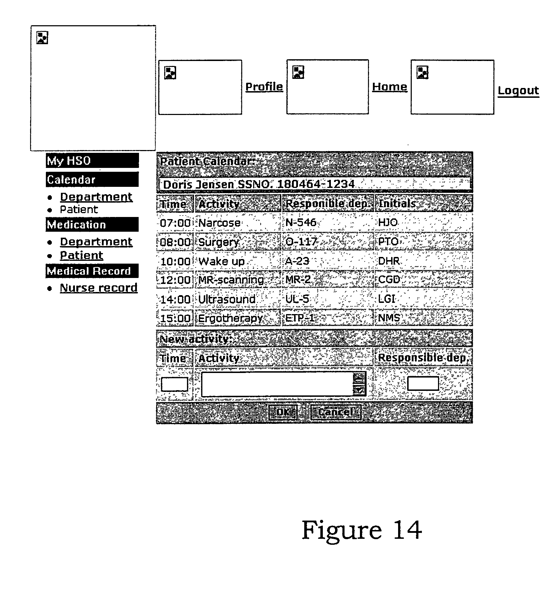

   Figure 14 is a departmental calender;

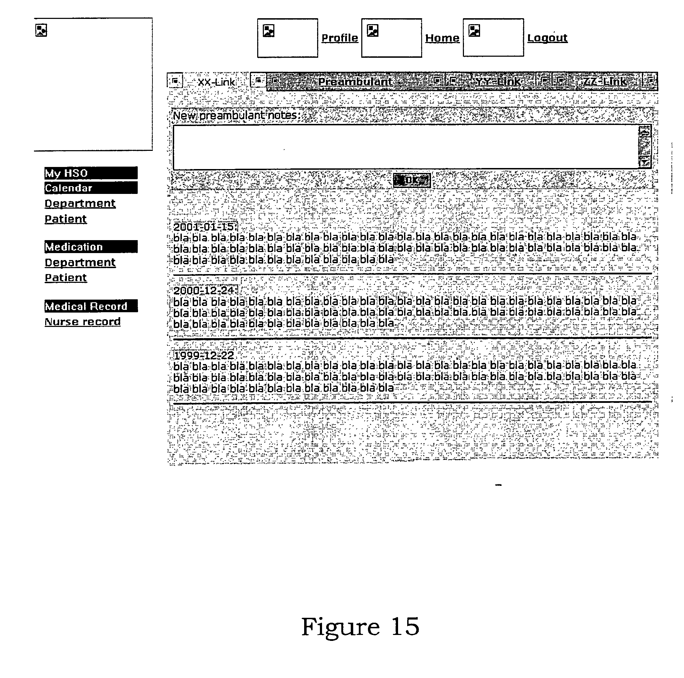

   Figure 15 is a patient specific screen;

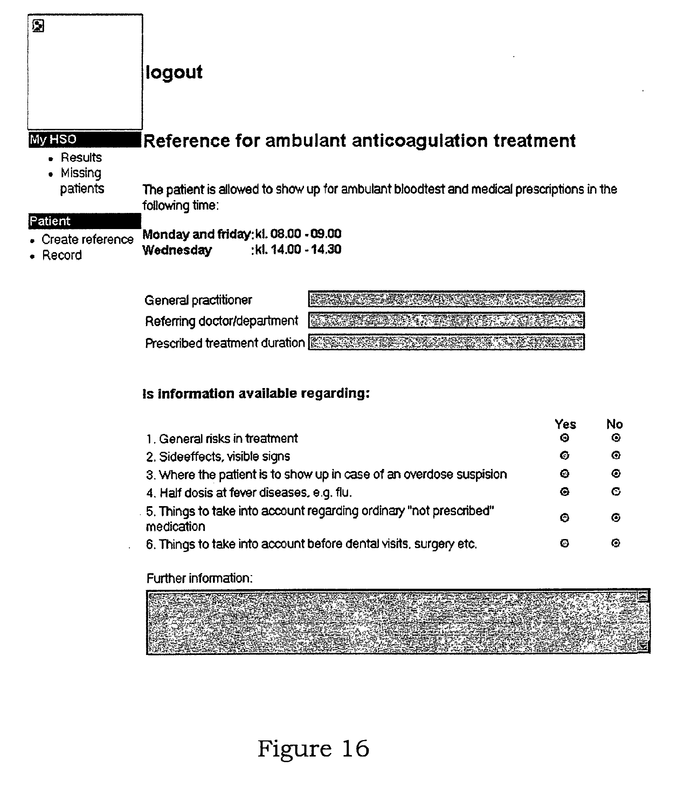

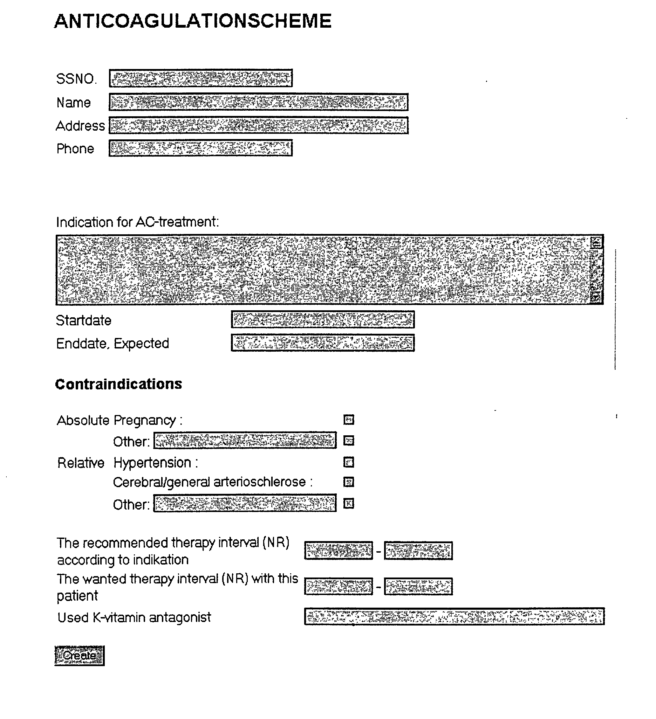

   Figure 16 is a screen for anticoagulant treatment;

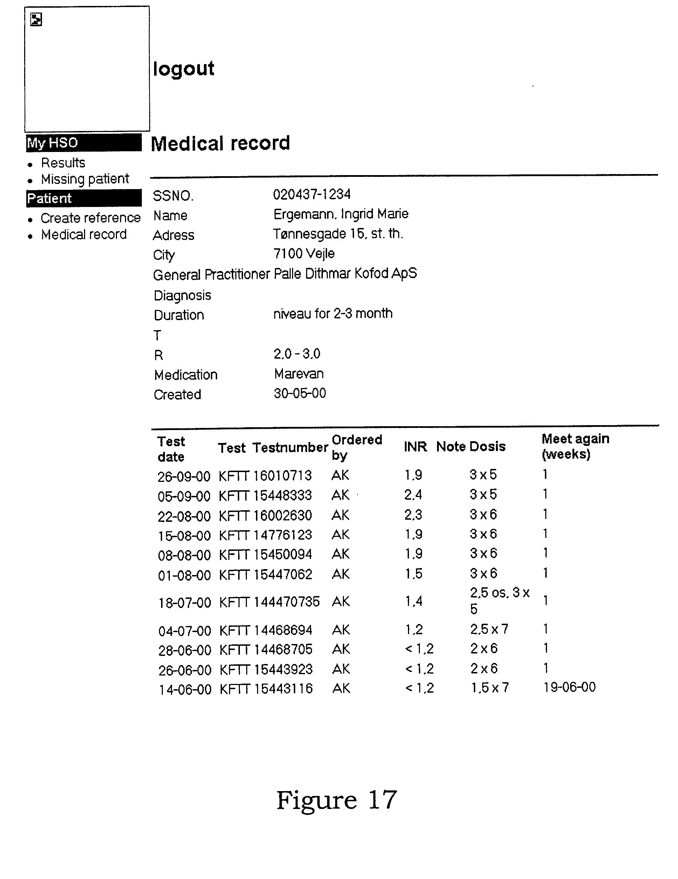

   Figure 17 is a patient specific journal;

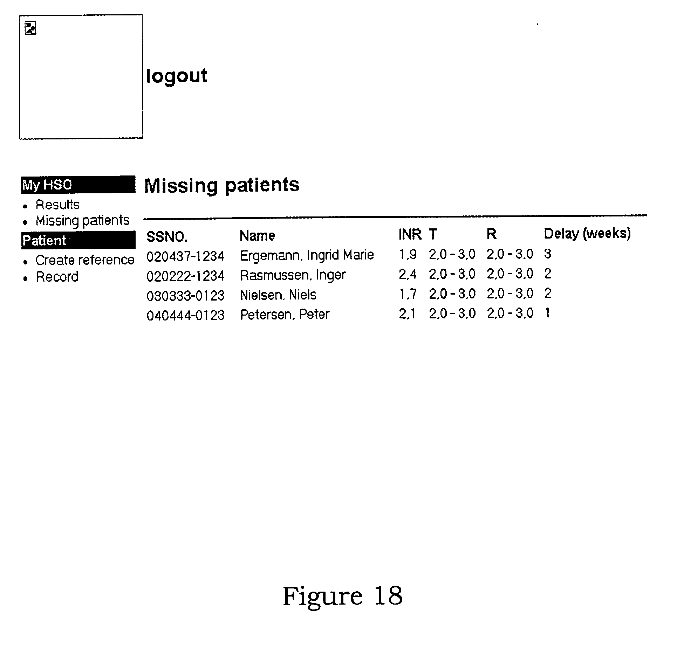

   Figure 18 shows a screen of patients who have missed, or not made appointments;

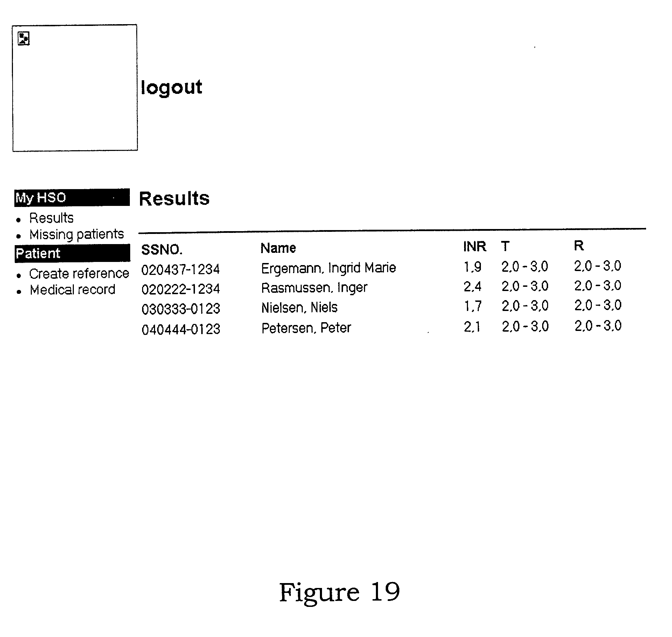

   Figure 19 shows test results for a group of patients;

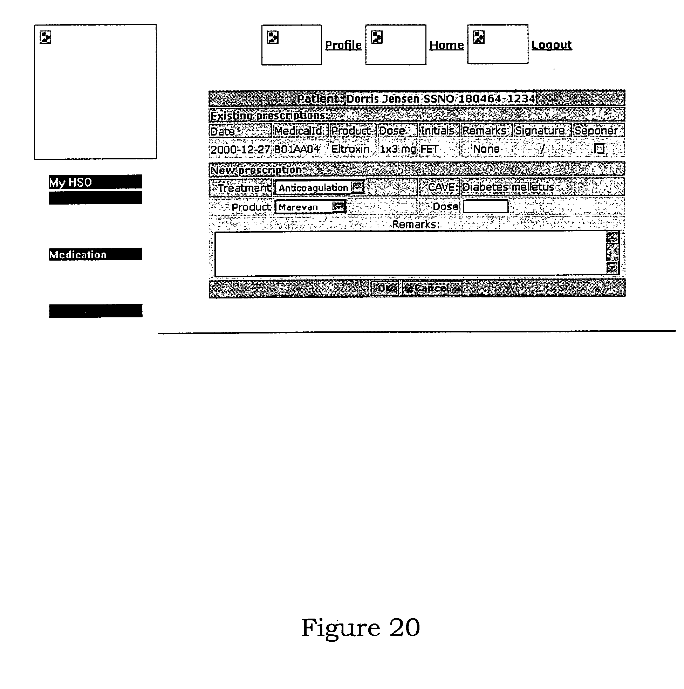

   Figure 20 shows a treatment regime for a specific patient;

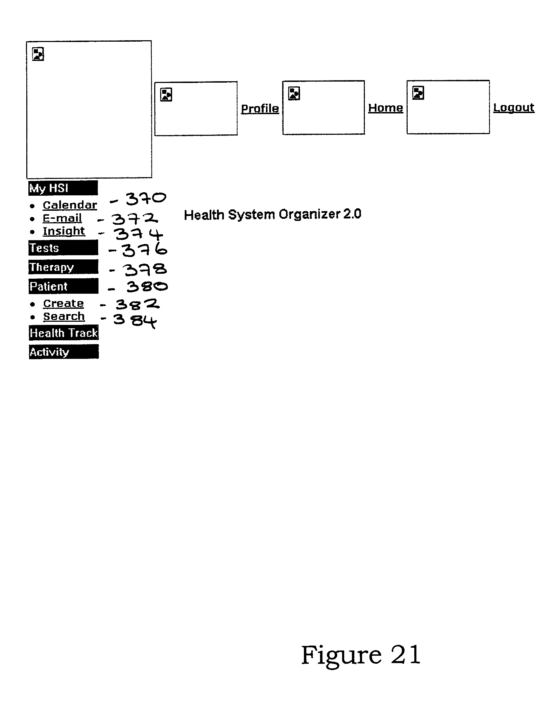

   Figure 21 is a front screen of a health system organiser;

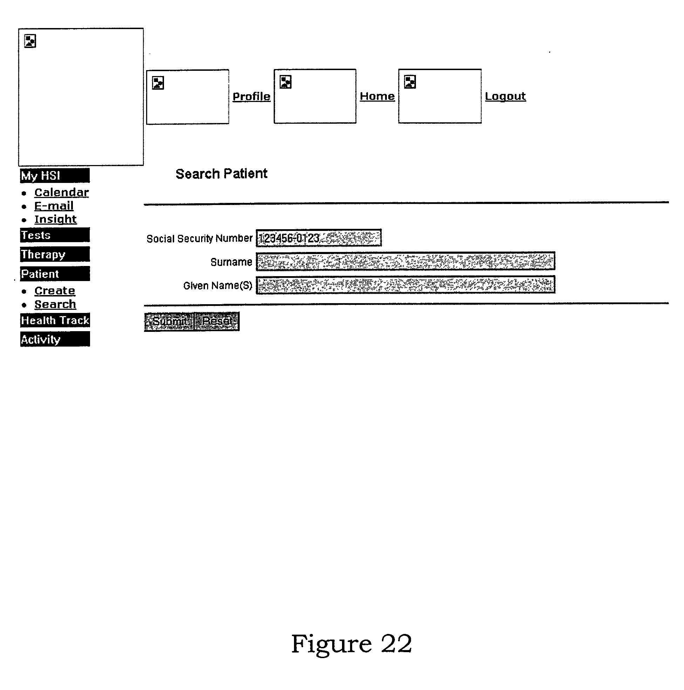

   Figure 22 is a patient search screen;

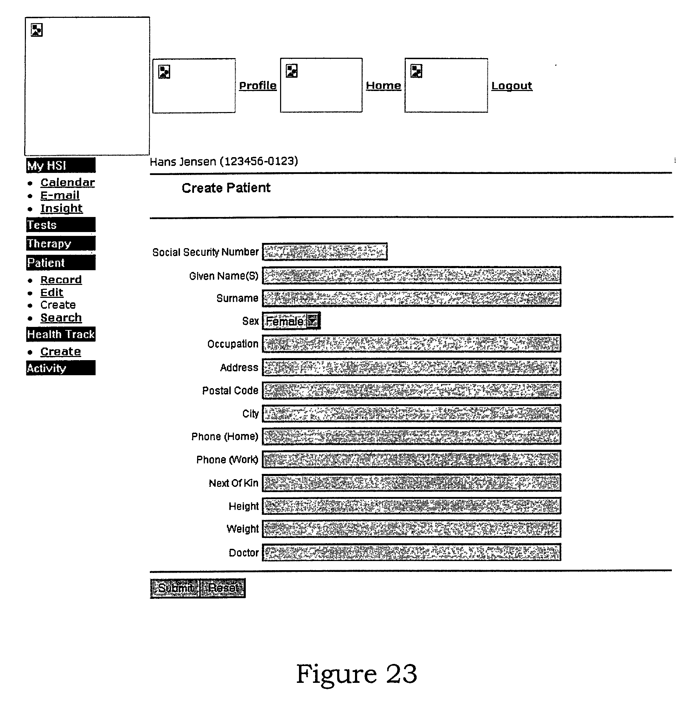

   Figure 23 is a new patient screen for creating a new health track;

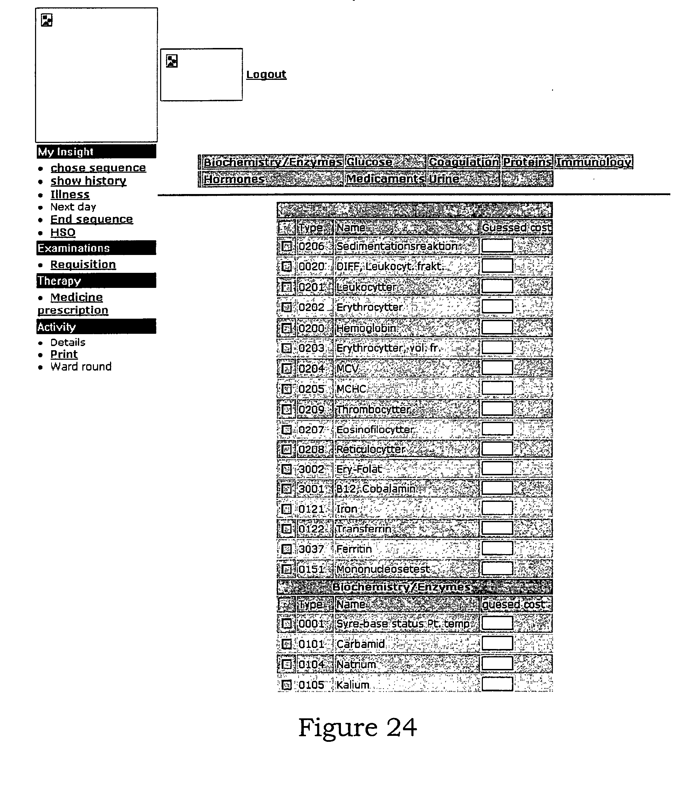

   Figure 24 is a test ordering screen;

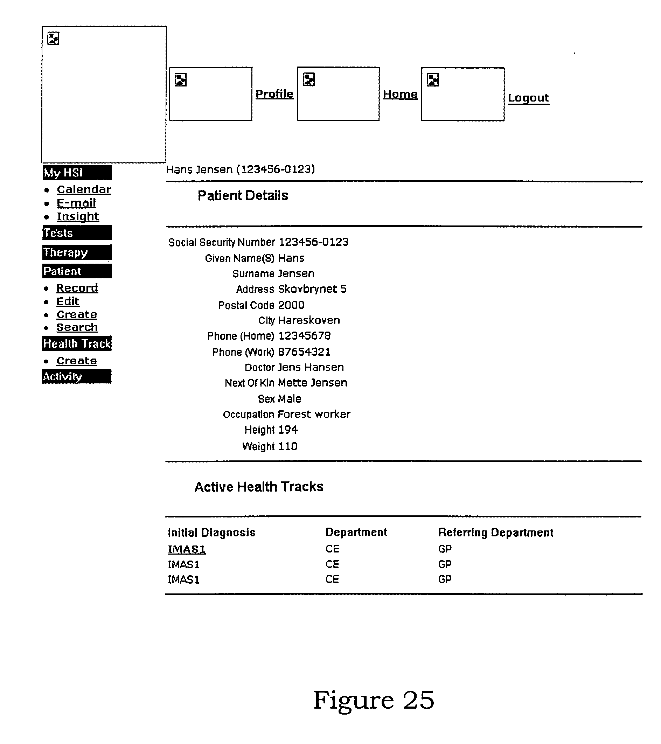

Figure 25 is a patient detail screen showing health tracks; and

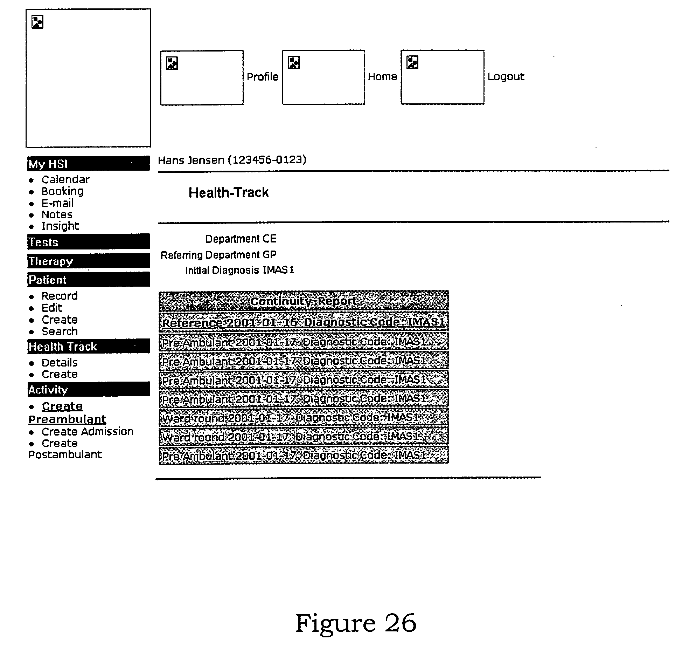

Figure 26 is a health track for a given patient.

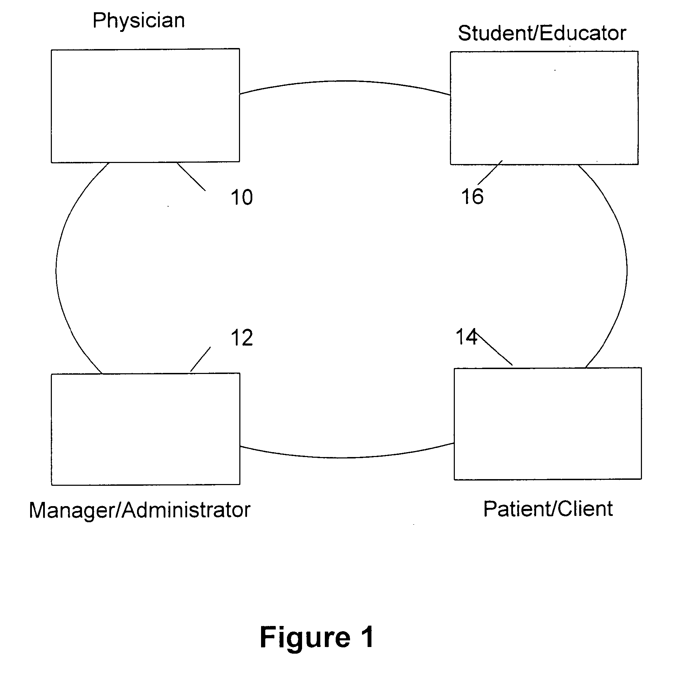

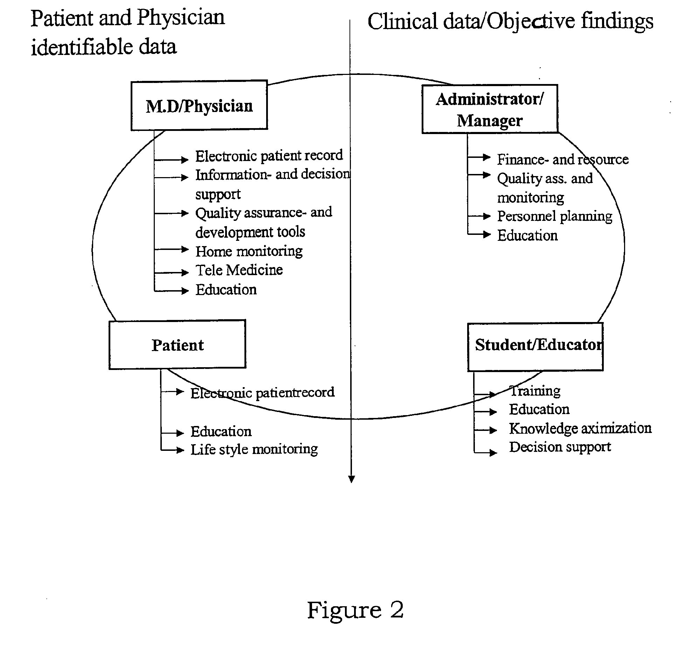

Referring now to Figures 1 and 2, the system embodying the present invention links the four key components of the healthcare marketplace and provides a secure infrastructure that solves the problems discussed earlier and leads to both a qualitative and quantitative improvement in healthcare provision. The system includes many enhancements over current software available in portions of the healthcare marketplace but has the advantage that all information is secure and accessible through standard Internet browsers. This enables the system to be installed without the need to replace current hardware and software systems. The system is structured so that individuals or each linked party is not required to change the manner in which they work, which is an important consideration in the medical profession which is conservative in nature.

As can be seen from Figures 1 and 2, the system links physicians or doctors, 10; administrators or managers, 12; patients or clients, 14; and students or educators, 16. This enables a wide range of functionality and advantages to be achieved which is not available with existing systems. This may be summarised as follows:

### 1. Education and Research

In the education and research field, information can be provided in real time from doctors and patients which protects patient and doctor confidentiality and yields qualitative and quantitative statistical information for education and research purposes. This information can be provided by removing from the medical data any reference to the patient or doctor so that an actual medical record becomes, to the educational or research establishment, a mere piece of information. However, it is provided in real time adding greatly to its usefulness.

Doctors can obtain an instant historical review of their diagnostic and treatment decisions and their impact on patient care. This enables doctors to evaluate the quality of their decisions and make improvements.

Doctors can also have a real time link with the medical educational establishment.

In turn, medical education can be adjusted and improved rapidly as can be the distribution of up to date information to students and practising physicians.

### 2. Patient Care.

The system enables the achievement of a continuous improvement in the quality of medical care for patients. Doctors have instant access to individual and private diagnostic and treatment second opinions by doctors leading to a reduction in the number of "mistakes" made in diagnosis and treatment.

### 3. Administrative and Financial Effectiveness.

Healthcare may be managed more efficiently and cost efficiently and a completely integrated suite of tools provided to manage all aspects of a hospital, doctors surgery or other healthcare provider.

### 4. Impact of the Total Healthcare System.

Of the above mentioned available functionality, the instant access to real time diagnosis and treatment data by educational and research establishments; the access by doctors and patients of up to date information on any disease, diagnostic or treatment technique; and the access to individual medical records without record loss, all have the potential greatly to impact on the total healthcare system by improving the quality of care delivered and reducing the cost.

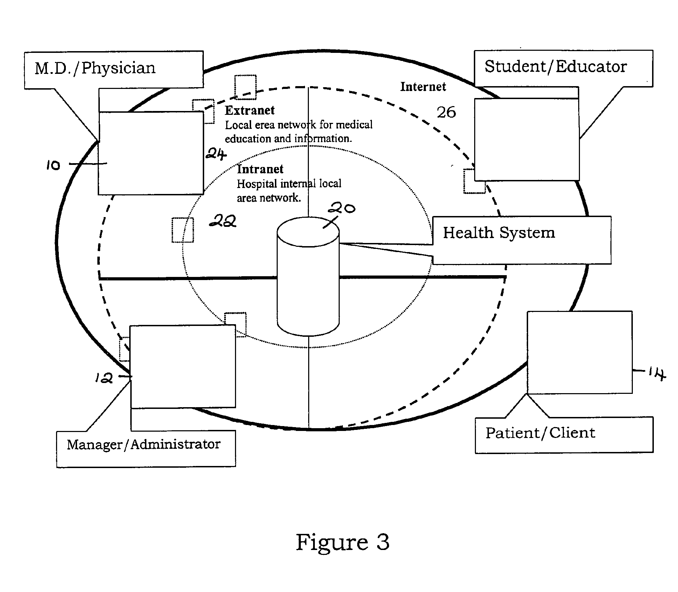

Figure 3 is a flowchart showing each of the four key areas and their relative interaction, along with their linkage through various networks such as the Internet, Intranet within a hospital and Extranet within a local Community and education centres. Thus, in Figure 3, the healthcare system 20 connects all four of the healthcare areas: doctors 10; administrators 12; patients 14; and students/educators 16.

The connectors may be through any convenient network including Intranet 22 for a hospitals internal local area network; Extranet 24 providing a local area network for medical education and information, and Internet 26, for example for retrieving information from outside the healthcare providers region.

The benefits that such a system can provide may be summarised as follows:
- Managers/Administrators/- Health authorities:: Cost savings, efficient delivery of health care.
- Doctors: Instant electronic second opinion, increased knowledge base, fewer diagnostic and treatment errors, real time training and instant record access.
- Patients: Security, safety and assurance.
- Teaching Hospitals and Universities: Faster and Cheaper real time links to the medical marketplace for education and research purposes, a distribution channel for education and training, faster improvements in medical education.
- Corporations (Pharma and Insurance): Faster and cheaper access to statistical real time medical field data for research and product development.

The embodiment described, may be viewed as a portal which combines existing and new software into a single user interface.

Existing hardware and software in user facilities such as hospitals need not be integrated. All common applications may be integrated enabling point to point integration with different applications. This gives a central solution with a single entrance to the entire internal IT environment for an organisation.

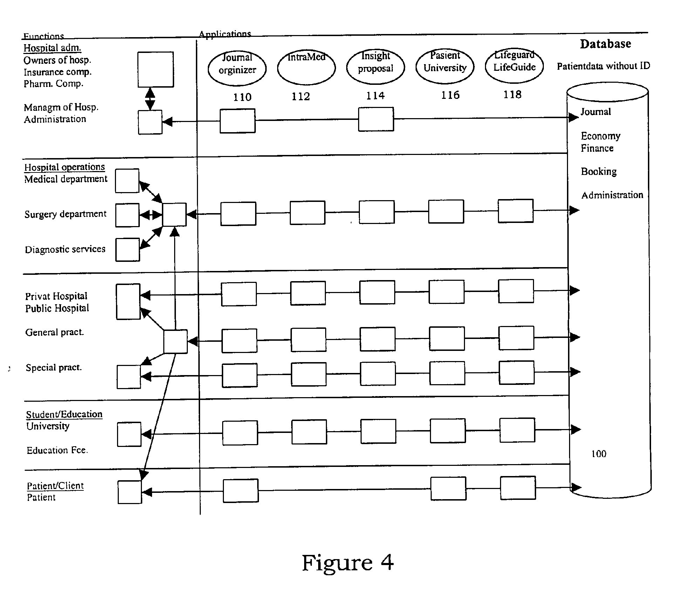

Figure 4 illustrates various applications which may be run under the system. All of these rely on the database 100 which is a repository of patient data without the identity of the patient or doctor. In other words, it is a repository of anonymous statistical patient information.

The applications running under the system are as follows:

Journal Organiser 110. This is an application which provides a medical reporting facility and can incorporate daily work in a single admission. It contains a basic structure that includes all the needs from the various medical specialities. It is a combined user interface and requirements system and it is Internet compatible.

Intellimed 112 may preferably be a second generation neural networking system which collects actual clinical information and compares it with the total knowledge in the central database 100. It allows the user to get second opinions from local, regional or international patient databases. Thus, it enables patient data to be retrieved from sources outside database 100. It will be appreciated from the above that all information is served to the user anonymously with respect to the patients identity and in a secure fashion.

Insight 114 is a virtual day-to-day workplace for doctors, nurses and other healthcare staff. It contains every possible action in a hospital from investigations to treatment and gives departments the ability to incorporate new procedures in a virtual manner before they are implemented in the hospital.

Proposal 114 is a software application which is an educational and implementational program for general practitioners. It contains a virtual clinic containing all possible daily activities for a clinic at work and provides a real time educational link with the external medical training environment.

Patient University 116 provides on-line contact, for example via the Internet, with the doctor. Both patient and doctor can monitor the treatment of diseases and other medical conditions if the patient is treated at home.

LifeGuard & LifeGuide 118. LifeGuard is based on a connection on-line with a patient's record. It is updated with the latest laboratory results and gives the patient the ability to explore a multitude of treatment possibilities and create simulations that examine the links between a cure and his own and other relevant statistical lifetime probabilities. LifeGuard is also an on-line link to patient records, updating recording according to laboratory results. Through use of the application, the patient can recognise changes they can make in their lifestyle that are important. The application will automatically create and customise the appropriate diet including all amino acids, proteins, enzymes, lipids and carbohydrates that are important to fighting a particular disease or medical condition.

Figure 4 also shows which of these applications are available to each of the four parts of the healthcare system illustrated in Figure 1. Applications are available if that application is indicated by a shaded box on the line leading from the function to the database 100.

Thus, the applications are all available to all functions under the Hospital Operations and Student/Educator headings. Within a hospital, medical and surgical departments and diagnostic services all have access and the pathway includes a link to general practitioners. General practitioners are shown as linked also to public and private hospitals, specialist practitioners and patients.

Patients have access to the journal organiser 110 and patient university 116 and, where national laws permit, to the LifeGuard and LifeGuide applications 118. Hospital administrators have access to the journal organiser 110 and Insight/Proposal applications 114.

It will be appreciated that the system requires the availability of patient data over the Internet. This raises issues of security. The system creates a strict divide between all personal identifying data and statistical data. If the validation of a person is acceptable, they may be given access to their own clinical data. It is not generally possible to see whom, the doctor and patient, owns the data, but a flag may be placed with information or questions and the next time the record is opened with the correct ID the information is automatically available.

The database or repository 100 serves as an information storage capability to preserve the information necessary to show a complete patient record. Its structure is complex and will be discussed later. The repository is designed to separate patient identifiable data from clinical data. This permits the ability to retrieve statistical data and analysis without comprising patient identification or confidentiality of specific information related to the patient. To provide maximum security regarding patient anonymity, personal data is stored in a separate part of the database structure which may be located on a physically different server. Thus, if one server was stolen, both personal and clinical data about patients would not be obtained.

The various components of the system will now be described in more detail.

The system uses standard Internet protocols and a functionality, in the preferred embodiment, based on Java 2 Enterprise Edition (J2EE). The architecture makes it possible to provide the doctor, or other healthcare provider, administrator, educator, or patient with extended functionality with no requirement on the client side other than an ordinary Internet Browser such as Internet Explorer V5 from Microsoft Corporation or Netscape Communicator V4 from Netscape Corporation. Any Internet enabled terminal may be used such as a WAP enabled mobile phone, PDAs, thin clients or PCs with a standard browser.

The system has been moved from the PC to the middle layer to avoid developing and maintaining several versions of client applications, leaving only the viewer on the client notes and increasing security. This enables each user to choose their own hardware and operating system architecture.

The system preferably only transfers text based HTML documents and compressed pictures, for example in GIF and JPEG formats. This is in contrast to most normal client server database applications which transfer vast amounts of data between server and client. The traffic is encrypted, for example using SSL. The core database communication run in a secure closed and high-speed connection between the application server and the database server. The database preferably runs on an RDBMS (Relational DataBase Management System) or any ANSI SQ6 compliant system such as 8I from Oracle Corp., DB2 from IBM Corp, Sybase, Informix or PostGreSqL.

The system servers are platform independent and run on UNIX versions commonly supported by major software vendors. A suitable server is Sun Solaris from Sun Microsystems Inc.

The system consists of three logical layers although the number of physical layers is immaterial. The layers are as follows:

1. The client or front end layer. This is a graphical user interface (GUI) and is preferably based wholly on open standards, making it possible to use a standard browser as the client application, thereby reducing the complexity of the client workplace, which may be a PC or thin client such as SunRay from Sun Microsystems, Inc. A browser must be installed, such as Netscape Navigator 4 or Internet Explorer 5. The front end uses HTML, Java Servlets, Java Server Pages (JSP) and XML/XSLT.

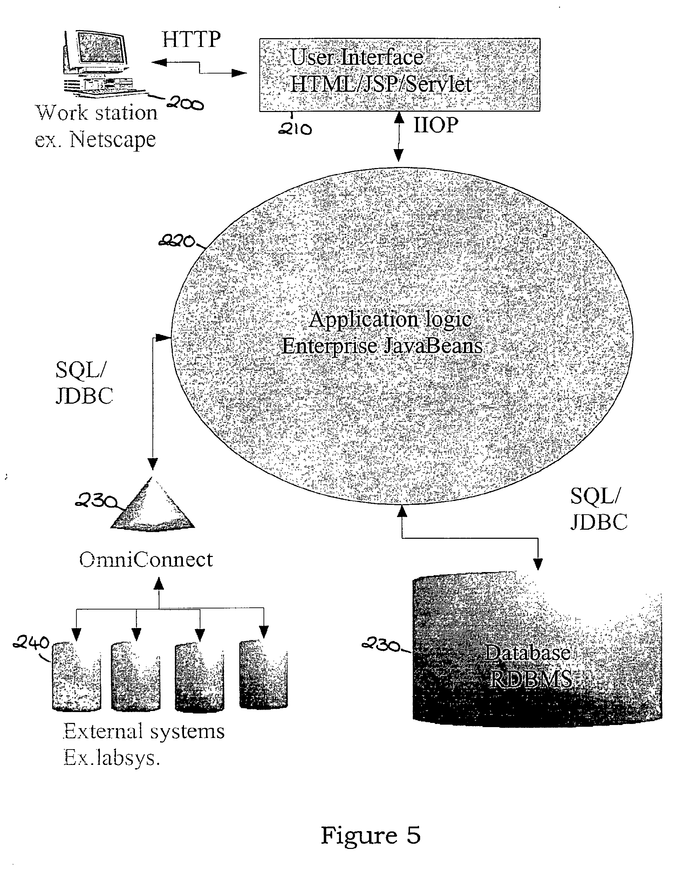

The logical configuration is shown in Figure 5. The user workstation is shown at 200 and the user interface at 210.

Where fat client software is used, the fat client is a swing based Java application which includes all the usual functionality of client programs such as keyboard short cuts and dropdown menus. The fat client us an XML browser which is downloaded and started when the user accesses a specific URL in their standard browser. The user downloads the fat client on their first access. Afterwards only updates and fixes need be downloaded. The only major difference from the thin client solution is that it has a rich functionality of common applications and is not limited to the capabilities of HTML. The speed will depend on the speed of the PC on which it resides. The thin client resides on the serverside and is accessed via a standard browser. In that case, nothing is installed or executed by the client machine, except for the browser, making it possible to use older or cheaper client computers as they only have to run a browser. Both types of client send and receive data using HyperText Transfer Protocol (HTTP) which is lightweight by nature making it possible for users to work with the client software of either type even with a limited bandwidth, for example using a modem.

The second layer is the application logic 220. This is the core of the system which is based on Enterprise JavaBeans using XML (extensible markup language) for input and output, making third party integration easy to perform as the Enterprise JavaBeans are an open standard component. The application or core layer performs all the computation. It receives requests from the front end and sends responses back when output is available. It is preferably built on a Java II Enterprise Edition (J2EE) platform using Enterprise Edition JDBC, Servlets JSP, XML and XSLT and other APIs from the J2EE platform. JavaBeans are standard component models. They give you the specification your components must fulfill, such as how the interfaces to the world appear.

The third logical layer is the data repository 230 which is a relational database implemented as a standard RDBMS and, in the preferred embodiment, is designed to meet the SQL 92 standard: ISO/IEC 9075:1992. The repository is a set of relational tables, sequences and views.

As can be seen from Figure 5, the application logic is also connectable to various external systems 240 through a universal connector 230. These external systems may include laboratory systems, hospital legacy systems etc.

The front end of the system appears to the user as a world wide web home page. However, instead of being a fixed HTML document it is generated dynamically based on a number of different parameters such as current user's work progress. This is intended to increase efficiency. The front end for a given client is not finalised until an analysis in cooperation with user's staff has been conducted. A default layout of the front end may be used.

The core of the system, the application logic, does not interact directly with the user. Large amounts of information have to be exchanged between the front end and different repositories. Part of this information has to be computerised into an answer to a user's request, or altered to keep repositories consistent. Moreover, a third party system may ask for information which must be retrieved from the appropriate repository and returned in a format such as XML. All functionality is handled in the application layer.

The core will service information to and from many other repositories as well as the system core. Many hospitals already have systems that solve specialised tasks. For example, some hospitals maintain patients' social data, using old legacy systems. Some solicit patients' addresses from a central location.

The core always collects its information from the repository where that information is maintained. As hospitals typically have different systems already in place, the repository must be able to maintain all aspects of the information needed in the core. Over time, the users can shut down their old legacy system and keep all data in the repository. As the repository is ANSI SQL compliant data can easily be retrieved even without using the core application.

Third party products, and the user interface call the application layer using IIOP protocol as the application layer is implemented using Java 2 Enterprise Edition technology. Customers can integrate third party software products in a seamless straightforward manner.

The core layer 220 implements the business logic of the domain model which describes the health care sector. The core layer consists of Enterprise javabeans of different types which will be described in detail. The core layer has the basic function of accepting requesting from the frontend or a 3^{rd} party application in the form of a XML document, processing the request eg. By retrieving information from the repository, applying some algorithm, and finally sending a reply back to the calling application eg. the frontend, in the form of a XML document.

The core application itself is divided into two different layers which perform different operations.

The first layer is the core Session layer. This layer is the layer that implements session specific logic. It consists of Session beans which are a special type of Enterprise Javabeans which do not reflect persistent data. They are used to perform a specific action such as retrieving a patient record. Since an entire patient record is a collection of information, all health tracks, all activities and all actions performed within those activities. When someone wants to view an electronic record, a session bean must retrieve the information included in that particular record for the entity beans included.

The system structures its information in the following way. A patient is an entity who is the owner of health tracks.

A health track is an entity which relates to one and only one patient, it provides information that is relevant for that particular health track, like referring department/doctor, CAVE (earlier diseases one must take into account), timestamp of creation, and the department which "owns" the health track. An activity is an entity which relates to one and one only health track (hence one patient), it keeps the information about that particular activity like notes, timestamps, labtests performed, medical prescribed etc. In the real world an activity could for instance be a pre out treatment. If it is a Pre out treatment, all the medicines prescribed, the labtests ordered and the notes, written inside that particular treatment are kept in one instance of an activity, which relates to one health track, which relates to one patient.

To get a full patient record a session bean must instantiate the patient entity, the health track entities related to this patient, and the activity entities related to the health tracks. A session bean is used to put the elements together.

Health tracks are related to one and only one patient based on a GUID (Globally unique ID) the patient is given when the patient entity is created. This GUID is the key used to identify the patient within the repository. The GUID does not provide any information about the patient itself, in order to retrieve such information one must have privileged to look in the patient specific part of the repository. A privilege, which is only give to the internal user (the system itself). Hence anyone can be allowed to look in the clinical part of the repository since no personal ID other than the anonymous GUID is kept there. The same scenario is relevant for the clinician.

If a user access the repository in a legal way through the Core, the GUID will be removed before the user sees the information - the social security number or other real-world ids may be viewable depending on the privilege of the user looking at the information.

The second layer is the core entity later. The core entity layer reflects the real work entities like patients, health tracks and activities. An instance of eg. a patient, holds all the information about that particular patient, and provides basic functionality relevant to that user like returning the information to a calling session bean. Thus, the entity layer reflects persistent data.

This layer at a logical level resembles a set of building bricks which are assembled by the session layer.

The core layer accepts input from the outside world in the form of XML documents. It produces output to the outside world in XML documents.

To retrieve its information, the Core layer uses two different scenarios, a Query Processor and Data Access Objects (DAO).

The Query Processor accepts a query from the core layer eg. to retrieve information about a patient. The core layer does not know where the information resides, if it is inside the repository or in a 3^{rd} party system, it must ask the query processor instead of searching all the possible locations for the document, which would be very time consuming. The Query Processor retrieves an XML document which describes where to get the information, and in what format the query is to be executed eg. SQL. A load.xml file is included which is an actual query process load.xml document. It then retrieves the information based on the information in the load.xml file, which must be configured if the repository is not used, and returns it to the calling bean.

The data access objects perform the same action as the query processor does, but in a different way. They do not use XML but are hardcoded in Java for performance and technical reasons. At a logical level the two different ways of retrieving information are the same.

The main advantage of allowing the core layer to retrieve its information from a data access layer, whether it is a query processor or data access objects, is that every installation will be the same except for the data access layer which makes it a lot easier to support lots of different installations. The core layer will always be implemented the same way no matter where the installation is. Furthermore, it makes it possible for them to coexist with legacy systems, only the data access layer needs to know about which legacy systems are to be used. This is extremely important for a system which is to be integrated with a variety of existing systems in hospitals.

Thus, a data access layer is provided between the core processing layer and the databases, be they internal or external to the system. The data access layer is encoded with details of what data is stored in what database. Thus, queries are sent to the data access layer from the core. The data access layer has knowledge of where to find the information and will retrieve it and return it to the core.

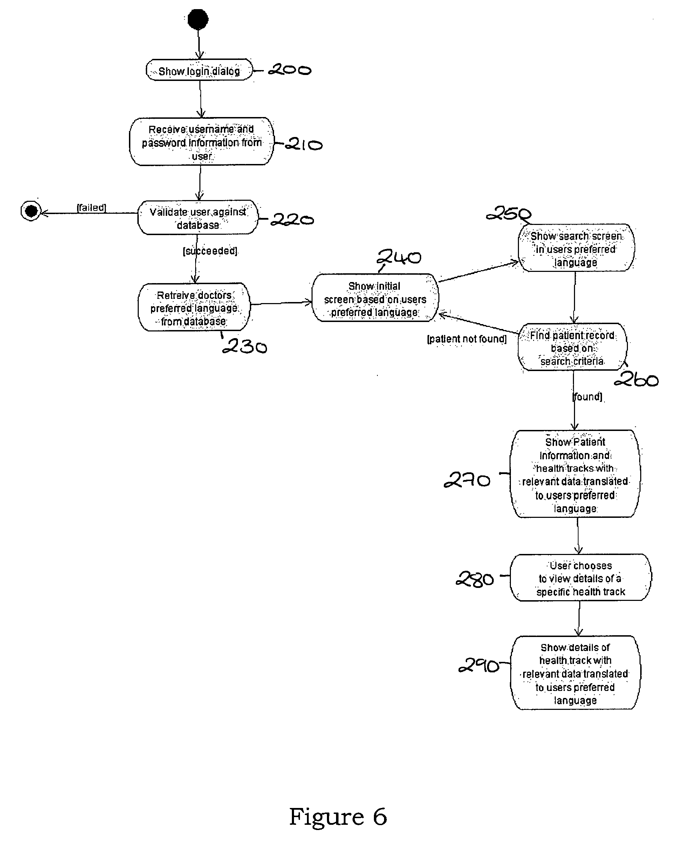

Figure 6 is a flow chart showing how a user can access the system.

At 200, the user is presented with a log-in dialog and at 210 the user provides their username and password. The username and password, as well as validating the user, tell the system what access rights that user has on the system. At step 220, the system validations the input username and password against values stored on a database, and, if successful, at 230, retrieve's the user, in this case a doctor, language preference from a database. At 240, the initial screen is displayed based on the user's preferred language. At 250, in response to a prompt from the user, a screen is displayed which allows the doctor to search in their preferred language. Once search criteria have been input, patient records can be found at 260./ if not found, the user is returned to the initial screen. If the record is found, the patient information and health tracks are displayed at 270 with the relevant data translated into the user's preferred language. At 280 the user chooses to view a specific health track and at 290 the user is shown that health track with the relevant data translated into his preferred language.

### Application Layer - Discussion of Applications

The functionality of some of the applications in the application layer will now be described in more detail.

### Journal Organiser

The Journal Organiser is a journal module used to register or display a patient's administrative data and to register data in concrete illness developments. All current as well as previous illnesses can be displayed. The user interface for the Journal Organiser is a standard browser based Intranet connection. Patient administrative data can be obtained either from existing patient administrative databases within the organisation or be registered directly in the system database (230 - Figure 5). Data is displayed in a manner that makes it impossible for the user to know whether data has come from an external source or been registered directly. Data relating to the development of an individual illness is shown based on development. Activity carried our during development is registered in code and text related to a text field filled by the actual therapist. In addition to each activity, the identity of the person that has made the registration is logged together with the time of logging. This data is available for all future displays of the activity.

The development of an illness will consist of a number of different pieces of information. This information will be displayed in the user infrastructure which is prepared according to the specific wishes of the department and the individual specialised groups, representing present or desirable future working routines with attached information combinations. Access to the information about the patient and illness development is protected by username and passwords. These are defined in respect of each individual user or institution's requirements and legal requirements.

Access can also be granted to objective clinical data which is also protected by username and password. The condition for access to this data is an indication from the therapist that the patient has given his consent that the objective clinical data may be used for scientific purposes. This data is only available without an identification of the therapist or the patient to which it relates.

### Journal Functions

The journal has the following functionality.

Information regarding the patient is split into two parts and stored separately:
a) Social information such as name, address, telephone number, next of kin, religion and personal information including weight, height, hereditary diseases, allergies etc.
b) Clinical information: current and previous illnesses.

Journal notes are sequence based and filed in the database making it possible to extract part data in any desired combination. Data can be effected to or from other departments, hospitals, general practitioners, specialists, therapists, etc.

The journal structure is similar to a traditional paper journal in that a journal heading is provided together with sections for pre out-patient, continuation, discharge, commentary, and post out-patient. A survey of the entire development of an illness can be generated.

A calender contains arranged current and future activities.

Video, stills, sound, amplitudes and text and any other multimedia may be filed.

A private note function is provided.

The printing, filing or mailing of standard letter electronically is available.

Reports may be generated electronically to national and international patient indices.

The journal organiser can be integrated into patient administrative systems, laboratory system, pharmacies, digital devices, home monitoring equipment, budget and economy systems and other IT systems.

Within the system, the journal organiser may be integrated through administration and prescriptive programs, booking systems, request and reply systems and cost monitoring systems.

The user interface will depend on the speciality required. A non exclusive list of possibility includes geriatrics, gynaecology and obstetrics, hepatology, haematology, infection medicine, cardiology, neurology, oncology, psychiatry and pediatrics.

### Medicine Administration Module

The medicine administration module may be integrated into a requisition/reply module. The program runs the normal precautionary checks made during daily clinic work, for example dosage frequency, interactions between medicines and warnings.

As with the journal organiser, the user interface is a standard browser based Intranet connection. This has facilities for the display and registration of pharmacy logistical data, department and pharmacy economy data/code systems together with patient administrative data and data connected to current or historic illness developments related to the patient in question.

The program allows the prescription of medicine using recognised drug indices and medicine codes. Generation of prescriptions may be made on consideration of a diagnosis code and indications to facilitate prescription routines. The prescription can be filled in automatically with the department's usual prescription for the actual diagnosis taking into consideration the rights of the therapist in question. A separate requisition program can receive the prescription and forward it to a pharmacy which will confirm receipt electronically. When the prescription has been made up, a message will be sent back to the journal to the effect that the prescription has been executed and will include confirmation of delivery, time and identity of the person responsible for making up the prescription.

A medicine prescription table shows what has been prescribed and what should be prescribed in future for an individual patient. For the nursing process, a prescription table may be produced giving an overview of the medicine prescribed for patients in the department. This module is capable of being integrated with existing packaged systems.

The module is capable of performing the following interactive functions:

Warnings. Special precautions can be secured for a given patient, for example reduced kidney function, liver function, allergy reactions etc.

Weight/Dosage. It can be ensured that the person's weight reacts to the prescribed dose as expected to avoid over or under dosage. The system can also prevent a given patient from being prescribed the same medication within the same day or other period from different parts of the healthcare system.

Drug Interactions. The system can ensure that drugs are not prescribed that will react with or will reduce the efficacy of other prescribed drugs so undermining treatment of the patient.

This user interface may be used for all the specialities referred to for the journal organiser. It is again emphasised that this is merely a selection of examples.

### Booking System.

The applications include a booking system which provides for automatic as well as manual booking of resources for patient examinations and treatments. The system allows overviews of current, potentially available and booked resources. The booking system is integrated into the journal organiser user interface described earlier. It is a standard browser based Intranet connection to specific resources in departments and out-patients' clinics. Bookings can be made from the user interface.

When a booking is made, the system can automatically book an appointment for pre-defined resources to take part in the specific activity. These may be physical or human resources. An automatic appointment may be changed manually or appointments may be booked manually in the first instance. Before allowing a booking to be entered, the system checks available personnel with the necessary qualifications considering work rotas, the availability of the relevant premises where these are required, which may consist of operating theatres and the like, and the availability of necessary equipment required for the task.

### Electronic Request Module

This is an auxiliary motor within the system making it possible to carry though electronic requests to external bodies such as pharmacies and laboratories. This module comprises an independent user interface and functions from the other modules described without the user being aware that it is an independent function.

The module functions as a server for the doctor who is diagnosing and treating an illness. This can be done either by providing supervision from specialities other than the one in which the doctor is working or by the provision of laboratory tests.

For example, a doctor working in geriatrics has access to supervision from anaesthesiology, neurology, departments of medicine, surgery and gynaecology. A doctor working in a cardiology department has access to the department of medicine, surgical and gynaecological departments. Similar cross links are available for all departments. As far as the latter is concerned, the doctor has access to laboratory tests such as from clinical biochemical, clinic genetic, microbiological, clinical physiological etc. departments.

Secondly, various therapeutic services are available such as surgical treatment, medical therapy and other therapies. Examples of surgical treatments include neurosurgery, oral surgery, vascular surgery and plastic surgery. Examples of medical therapy include neurology therapy ophthalmology therapy and psychiatric therapy and examples of other therapies include physiotherapy, neurological rehabilitation chiopody and dietetic therapy.

### Interactive Training Module.

Objective clinical patient data and economical data form a part of this module. The software is constructed such that a person is taught to follow current improvements in the manner in which problems are met and solved by playing against himself. The software is prepared locally in accordance with instructions and procedures with which the practitioner is expected to be familiar with or which it is desirable for staff to be taught. A responsible therapist selects the actual illness process to be used for training.

This module is used with a user interface such as the journal organiser from the environment in question. Thus, training is performed in an environment and with data with which the user is familiar.

The training can include objective tests, clinical laboratory tests, invasive tests, diagnosis economy, therapy, nursing and discharge.

### Neural Network and Expert System

The second generation neural network has been referred to earlier. It functions as a permanent support to decision making processes. Using the neural network and information contained in medical records, the module is able to indicate the likelihood of a given diagnosis based on sample replies and personal parameters.

This module may be accessed from an icon in the journal organiser and has two user interfaces. The first is a guide which is an expert system. This includes text book explanations and instructions or procedures that should be followed absolutely. The second is a test bench. Here, diagnoses are expressed as a likelihood based on the system's total amount of data or know how and only the next step is suggested. Diagnosis and relevant differential diagnoses appear as up to seven parallel horizontal bars in accordance with probability. For example, the most probable diagnosis may be shown as the longest and in a different colour from the rest.

This module may be used to support clinical tests such as clinical biochemistry, microbiology, physiology and nuclear medicine etc. and the recommendation of tests and therapeutic initiatives.

### Finance Module

This module monitors costs against budget and registers all costs of a department provided that a cost program connected to the desired activities has been installed into the system. The costs can be reported directly to the administrative function which can then see a departments expenditure against budged in real time and make an entry into the finance system.

### Staff Module.

This module allows a complete differentiation between personal profiles for the use of salary calculations, holiday planning and all considerations promoting the individual employee's cooperativeness.

### Remote Transportation Module.

This module is used to transport pictures, sound and text and may be integrated with all tele-medicine programmes.

### Patient Monitor.

This module, referred to previously as Life Guard (TM) enables a team approach to be adopted between doctor and patient. It is integrated into the journal organiser and the patient's user interface. It functions to observe trends and sudden changes in laboratory values. The module announces itself in a pop-up window to both doctor and patient with information that the necessary initiatives which should be occasioned by the observed trends and changes. Thus, the module acts on the patient/user interface as a surveillance and warning about deficiencies in existing treatment which could inconvenience or endanger the patient. The system may include sound, graphics and text allowing both partially sighted and hearing impaired patients to use it.

It can function for clinical tests, such as clinical biochemistry, microbiology, physiology, nuclear medicine etc., consumer goods such as utensils and support such as providing automatic time check contact to out-patient's clinics, general practitioners, home care etc.

### Life Style Module

This module, earlier referred to as Life Guide also aims to optimise the relationship between doctor and patient and is integrated into the journal organiser and the patients' user interface. However, the purpose of the module is to suggest changes to lifestyle as well as life quality improving measures such as dietary changes and exercise habits. These suggestions are based on the present patient situation registered in the Life Guard module. The module informs about appropriate changes to patient's lifestyle, medicine intake and can inform the doctor treating the patient, for example by a pop-up text.

The functionality of the module encompasses clinical tests, commodities such as suggestions for menus and purchase of favourite commodities including interaction with local suppliers and support, providing automatic contact to out-patients clinics, general practitioners, home care etc.

### Patient Education Module

This module, referred to earlier as patient university (TM) improves the relationship between the actual treatment and the patients observance of the doctor's recommendation. The programme establishes a direct contact between the two via the Internet. It allows the patient to read about his own disease in relation to his present status and can be used, on recommendation from a doctor as an educational facility to the treatment options of the patients disease.

### Interactive Clinical Module

This module surveys all the procedures within a healthcare provider for which instructions are prepared such as a diagnosis, therapy, laboratory tests etc. This module also uses the second generation neural network making it possible to check a large number of facts without having to disturb a doctor. The manual only informs if apparent mistakes are made and always relates to the instructional text and recommended literature. The manual is integrated into the journal organiser user interface and is a standard browser based Intranet connection to all departments and out-patient's clinics in a given geographical area attached to the actual patients working diagnosis. Internet access is provided via local connections. The user interface is protected by means of local log in requests and from this interface all relevant procedures can be integrated into the neural network. The module respects a given therapist's professional competence according to the profile of that therapist provided by the log in.

Thus, the functionality of the module is to provide a clinical manual to monitor handling processes such as objective tests, clinical laboratory tests, invasive examinations, therapy, nursing and discharge.

### Patient/Relative Module.

The patient is defined as a person with an objectively recognised condition, verified by a doctor, for whom a medical record has been or will be created in the journal organiser or in a similar system that can be integrated into the overall system. The exception to this is a normal pregnancy in which case a specially integrated pregnancy record is created for the midwife. A new born baby is considered as a patient within the system as an entirely new record process will be created.

The patient/relative user interface is a standard browser based solution allowing display of all health/illness data related to the patient. The user interface can be prepared in accordance with special needs, in case of special illnesses, for example, types of diabetes, rheumatic diseases, etc. The function of the interface is to give an easily readable view of the patients/relatives contact details and prepare illness information. The user interface has two parts, one for the patient and one for the relatives. The module can be integrated with the LifeGuard, LifeGuide and patient university modules referred to above.

### Clinician/Therapist Module

A clinician is defined as the person who is administering therapy and is usually qualified by an exam and subsequent authorisation. Exceptionally this may be a person temporarily authorised to act, for example in the case of a disaster. This module is one of the four basic user interfaces within the system and is a standard browser based Intranet connection to all health/illness data relating to an actual patient. The interface is protected by username and password and from the interface the clinician can carry out all entry functions as well as being able to check surveys of illness developments. From this, requests and replies can be made and received together with booking of appointments and the like. The module is integrated with the journal organiser, the request/reply module, booking module, support function module and education and training module.

### Administration/Management Module

The administrator is defined as any person whose task is to analyse the result of a patients dealings with the healthcare system in order to be able to vary the administrative processes such as economics, personnel, time spent and building administration. This module is one of the four basic user interfaces within the system. It is a standard browser Intranet connection to all health/illness data belonging to the actual institution which is to be analysed. Access is granted only to anonymous data to preserve doctor and patient confidentiality. Through the user interface, the administrator can make searches and analysis in accordance with access levels permitted by their username and password. The interface can be integrated with the financial and staff modules, patient communication module and education and training module.

### Student Teacher Module.

This module defines a student as any person whose task it is to be a student or to take part as a student in education within the area of the systems professional areas. Teaching is across the four areas shown in Figure 1, patients/relatives, clinicians/therapists, administrators/leaders and the actual basis education of all professions working in these categories.

The user interface is one of the four basic interfaces within the system and is a standard browser based Intranet connection to all logistical data, economic data, DRG codes, anonymous personnel data, anonymous health/illness data related to the actual institution required for educational purposes. Entry is only given to anonymous data and via the interface the student or teacher can make searches and analyses allocated to a level determined by their username and password. This module integrates with the journal organiser which acts as the introductory model in which all entries in the system can be shown, be they historical data or illness development data. It is also integrated with the patient education, patient communication, decision support, Life Guard and Life Guide modules.

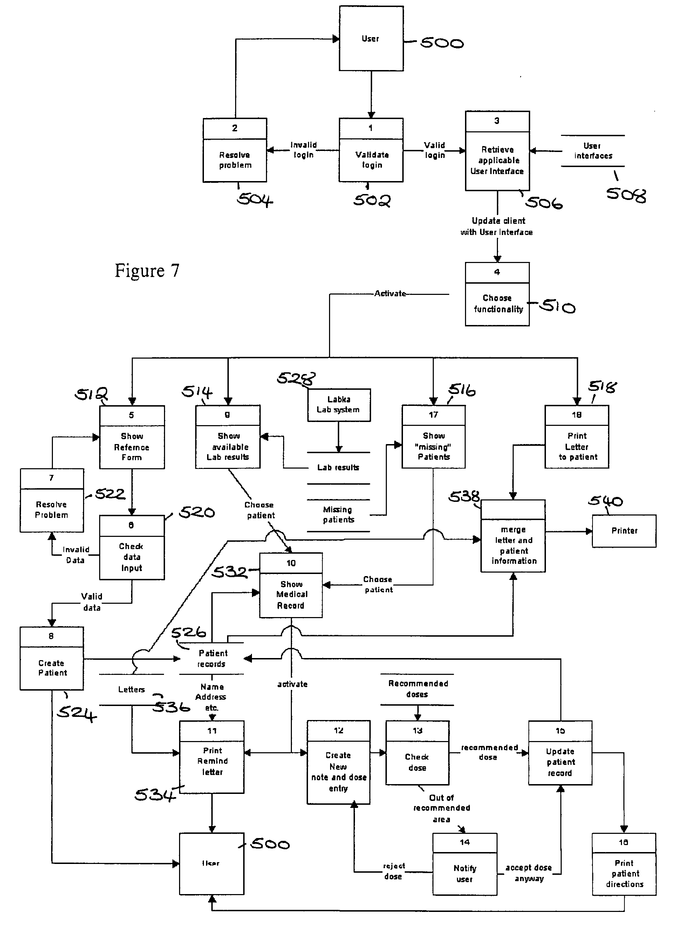

Figure 7 shows in more detail an overview of the system in operation. A user 500 logs into the system at 502. The user name and password are checked and if they do not match stored records the login is deemed invalid and the problem requires resolution at 504. If the login details are correct, the system will retrieve at 506 the applicable user interface from the system from the available interfaces. The actual interface retrieved will depend on the identity of the user and their degree of authority, which is encoded into their login details. The interface is retrieved from a store 508. The interface is sent to the user as an XML or HTML document and at 510, the user selects the functionality he requires from the options provided by the interface. In this example, there are four possibilities: 'Show reference form 512'; 'Show available lab results' 514; 'Show missing patients' 516; and 'Print letter to patient' 518. Each of these will be considered in turn.

If the user selects the 'Show reference form' option 512, he will be required to enter data into the form presented on screen. In this case it relates to the creation of a new patient record. At 520, the data entered is checked, for example to see whether any fields are missing. If the data is invalid, the process goes through a loop 522 back to the reference form to resolve the problem. If the data is valid, at 524 the system creates a new patient and sends the new patient data back to the user 500 and also to a store of patient records 526.

If the user selects the 'Show available lab results' option 514 he can select results 530 to be downloaded from a remote laboratory system 528 which may or may not be part of the present system but with which the system can communicate. The user may want test results for a specific patient in which case that patient is selected and the patient's medical record sent at 532 from the repository of patient records 526 to the user in XML or HTML format to be displayed in the user's browser. The user can then access test results for that patients from the lab results store 530.

If the user selects option 516 'Show missing patients', missing patient dat is retrieved from a store of missing patients which forms a part of the repository and displayed. The user can select any patient and display their medical records at 532.

If the user selects option 518 'Print letter to patient', letter information, for example standard letter formats etc. are retrieved at 538 from a letter store 536 and merged with patient information from the patients records store 526. The merged letters can be printed at a printer 540.

The patients records and letters stores can also be used by the user to print reminder letters, shown at 534 for example regarding forthcoming appointments. This is done with reference to the patient medical record. The user may also, from the patient medical record create new notes to enter on the patient record, at 542 and new drug dosage directions. Those dosage directions can be checked at 544 against stored details 546 of recommended doses. If within the recommended range, the patient record is updated at 548 and directions or a prescription are printed for the patient at 550. If outside the recommended range, the user is notified at 552 and may reject the dosage or accept it. In the case of the latter, the record is then updated and the dosage printed for the patient as before.

The foregoing will how be illustrated with reference to Figure 8 to 26 which show a number of screens which may be displayed to a user in the course of system use.

Figure 8 shows the opening screen with which the user is presented, once she or he has logged onto the system. Access rights are determined on a user by user basis and so different staff, at different levels of authority, will see different access screens.

In the figure 8 example, the screen will display, next to a logo, which may be unique to the hospital, four topics: Nurse care record 300, Anticoagulation treatment 302, Prescription details 304 and HSO (Health System Organiser) 306. By selecting one of those topics, the user will enter the relevant routine. It should be appreciated that this example shows how the system works for a specific anticoagulation treatment and that the screen is tailored accordingly. The four tracks available will now be described in turn.

At Figure 8, by selecting the nurse care record, the user is taken to the screen of Figure 9 which, around a main window 312 gives options for patient profile 310, Homepage 312 and logout 314. To the left of the window are three sets of options. The first 316 ties up with the main system calendar and offers department 318 and individual patients 320 calendars; the second 322 gives access to prescribed medicine information, again for a department 324 or individual patient 326. The third, 328 is the journal and has a single access to this nursing journal 330.

Figure 10 shows the nursing journal for a given patient identified by name (Doris Jensen) and social security number CPR 180464-1234. This screen has been access by selecting "nursingjournal" 330 in the pervious screen. The screen has the same menu options as the previous screen, but in the main window, displays patient notes in so far as they relate to the nursing care administered by that patient. Thus, the record shows on 5^{th} January 2001, a physician identified as EJB recorded diagnosis information 332 and on 9^{th} January, physician SAM recorded an operation description 334. There are further nursing notes 336 from KLM and ADE on 10^{th} and 11^{th} January together with a new window 338 for inclusion of new notes.

Figure 11 shows the prescription records for the patients in a department or ward, accessed by selecting the "department" link 324 from the menu. In the figure, details for three patients are shown. Doris Jensen 340, Jens A Madsen 342 and Gerda Frederiksen 346. In each case, the prescription is shown the dosage, the prescriber and details of when and how doses have been administered.

Figure 12 shows the patient prescription record 326. The example given is for the patient Doris Jensen. It will be seen that the details comprise the information on the department record of Figure 11 and a list of earlier treatments.

Figures 13 and 14 show, respectively, the patient and departmental diaries. Thus, Figure 13 lists the activities for patient Doris Jensen together with a reference such as a hospital room and includes space for new appointments to be entered. Figure 15 lists all the appointments for patients on the ward/department by time. The user may select any one of those patients to be taken to a patient specific screen as shown in Figure 15. This screen shows a list of previous notes relating to that patient and provides a window for entry of a new note.

Figures 16 to 19 show the screens that can be viewed under the anticoagulation treatment heading 302. It should be remembered that this is one of many treatments and in a less specialised environment may be first selected from a broad range of possibilities.

### The screen offers the user four choices:

Results, 350; Missing 352; create patient 354 and journal 356. The first two are available under the heading My HSO and the second two under the patient heading. The results heading gives a doctor access to results from laboratory tests which are supplied from a laboratory subsystem. This list is actually a stack to which lab results are appended. The doctor clicks on a patient to open the patient's medical record. He then analyses the latest results for that patient and compares it with past results. In the case of an INR test, for example, in which blood thickness is measured, the doctor will prescribe a course of medication and enter a note onto the record. The system will validate the dosage prescribed by reference to stored approved doses and according to the change in INR from the last test result. It also checks whether the INR is within is acceptable range. If the dose is not within the recommended range, the doctor is notified and is able to change the dose or include a note explaining why a dosage outside the recommended range has been prescribed.. The system will not prevent an apparently 'wrong' dose but will require the doctor to be made aware of what he is doing.

The 'missing' heading gives details of patients who have not turned up for appointments within a particular period. This may include patients who have missed specific appointments and patients who should have presented themselves within a given period. For example to monitor the progress of a treatments or condition. The create patient option allows the user to create a new patient record.

Figure 17 shows the journal that is displayed by selecting Journal link 356. It details patient information, diagnosis and treatment and lists the history of administration of treatment. In this respect it will be noted that the system operates by establishing a case track for each patient for each illness or condition, for example pregnancy, being treated.

Figure 18 shows a list of patients being treated who have not presented themselves for treatment and is available from the missing field on the previous screen. This information is not patient specific and is available through the health system organiser.

Figure 19 shows the test results for a number of patients. It will be seen that this screen contains links 360, 362 to laboratory information including the ability to view test data and results for any given patient.

Figure 20 shows the medication field 304 (Figure 8) which displays, for a given patient, prescription information and includes a field 364 for the treatment and a field 366 for the drug being administered. In this case an anticoagulant drug Marevan is being administered. A field 386 is included for dosage and a warning 370 is displayed alerting the practitioner that the patient is diabetic. That information is stored as an attribute with patient data. A field 372 is provided for the practitioner to insert remarks.

Figures 21 to 23 show screens from the main system organiser. This provides central functions which overlie many of the specific functions provided by the system. As can be seen from the figure, the user has access to calendar and e-mail facilities 370, 372 and Insight, the on-line educational aid and diagnosis assistant 374. The user can also order tests and therapies and, depending on how the system is configured, has access to a range of further functionality, including patient specific activity 380 which includes searching for a particular patient and creating a new patient.

Examples of patient creation and search screens are shown in Figures 22 and 23.

Figure 24 shows a portion of a test order form available under the tests heading. This allows a practitioner to order any test to be carried out. The figure only shows a small portion of the available tests which would normally be available to a physician.

By way of further example, Figure 25 shows a patient details screen which includes details of active health tracks; and Figure 26 shows an example of a health track.

It will be appreciated that the embodiments described can be varied in many ways without departing from the various aspects of the invention which are defined by the claims appended hereto.

## Claims

1. A healthcare management system, comprising: a plurality of user terminals;
an application layer coupled to the user terminals and running a plurality of healthcare related application programs;
a repository for holding patient data, the repository storing patient identification data and clinical data separately, whereby clinical data may be retrieved with or without the patient identification data to which it relates;
a data access layer arranged between the application layer and the repository for retrieving data from the repository or one of a plurality of further databases external to the system, the data access layer including information regarding the location of data required by the application programs.

2. A healthcare management system according to claim 1, wherein the data access layer comprises a query processor which receives requests for data from the application layer and includes means for retrieving a document describing where to find the data requested and means for retrieving that data and passing it to the application layer.

3. A healthcare management system according to claim 2, wherein the document describing where to find requested data also describes the format of that data.

4. A healthcare management system according to claim 1, wherein the data access layer comprises data access objects.

5. A healthcare system according to claim 1, 2, 3 or 4, wherein the application. later models patients as entities owning health track entities.

6. A healthcare system according to claim 5, wherein health track entities have associated activities.

7. A healthcare system according to claim 5 or 6, wherein application logic in the application layer is implemented using enterprise Java beans.

8. A healthcare system according to claim 7, wherein the enterprise Java beans include session beans for implementing session specific logic.

9. A healthcare system according to any preceding claim, wherein each of the user terminals includes a browser.

10. A healthcare system according to claim 9, wherein the terminals are fat client terminals.

11. A healthcare system according to claim 9, wherein the terminals are thin client terminals.

12. A healthcare management system comprising a computer network for communicating between a plurality of user terminals and an application system for running a plurality of application programs, the application system including a database storing clinical data and related patient data, and an interface to a plurality of further data stores each containing patient related information; the communications network linking medical practitioners, healthcare administrators, patients and educations establishments and/or students, whereby each have access at least to clinical data stored in the database.

13. A healthcare management system according to claim 12, wherein the patients and medical practitioners further have access to some or all of the related patient data stored in the database.

14. A healthcare management system according to claim 12 or 13, wherein the application programs include a program for giving medical practitioners medical options based on patient information entered by the practitioner.

15. A healthcare management system according to claim 12, 13 or 14, wherein the application programs include a program for recording patient medical history.

16. A healthcare management system according to any of claims 12 to 15, wherein the application programs include a program for requesting laboratory test data and the communications network includes a link with a laboratory to which a laboratory test request may be sent.

17. A healthcare management system according to any of claims 12 to 16, wherein the application programs include a medication administration program for recording the medication to be delivered to a given patient and recording the history of dosage administration.

18. A healthcare management system according to any of claims 12 to 17, wherein the application programs include an interactive training module.

19. A healthcare management according to claim 14, wherein the medical opinion programs interacts with a neural network to support the practitioner's decision making process.

20. A healthcare management system according to claim 18, wherein the neural network has a first interface comprising a practitioner guide.

21. A healthcare management system according to claim 19 or 20, wherein the neural network has a second interface comprising a test bench.
